Europäisches Patentamt

European Patent Office

Office européen des brevets

(19)

(11) Veröffentlichungsnummer: **0 138 232**
**B1**

(12)

# EUROPÄISCHE PATENTSCHRIFT

(45) Veröffentlichungstag der Patentschrift: **05.09.90**

(21) Anmeldenummer: **84112570.1**

(22) Anmeldetag: **18.10.84**

(51) Int. Cl.⁵: **A 61 K 6/10,** A 61 K 6/08

(54) **Masse zur Herstellung von plastischen bzw. harten Massen für dentaltechnische, (dental)medizinische und verwandte Zwecke, Verfahren zu deren Herstellung und Verwendung derselben.**

(30) Priorität: **19.10.83 DE 3337986**

(43) Veröffentlichungstag der Anmeldung:
**24.04.85 Patentblatt 85/17**

(45) Bekanntmachung des Hinweises auf die
Patenterteilung:
**05.09.90 Patentblatt 90/36**

(84) Benannte Vertragsstaaten:
**AT BE CH DE FR GB IT LI LU NL SE**

(56) Entgegenhaltungen:
**DE-A-1 620 891**
**FR-A-2 534 803**
**GB-A-2 102 290**
**US-A-3 558 540**
**US-A-4 155 890**

(73) Patentinhaber: Hofacker Freifrau von Nostitz,
**Frauke**
**Allescherstrasse 45**
**D-8000 München 71 (DE)**

(72) Erfinder: **Hofacker Freifrau von Nostitz, Frauke**
**Allescherstrasse 45**
**D-8000 München 71 (DE)**

(74) Vertreter: **Hansen, Bernd, Dr.rer.nat. et al**
**Hoffmann, Eitle & Partner Patentanwälte**
**Arabellastrasse 4 Postfach 81 04 20**
**D-8000 München 81 (DE)**

**Die Akte enthält technische Angaben, die nach dem Eingang der Anmeldung eingereicht wurden und die nicht in dieser Patentschrift enthalten sind.**

EP 0 138 232 B1

Courier Press, Leamington Spa, England.

**Beschreibung**

Die Erfindung betrifft eine Masse zur Herstellung von plastischen bzw. harten Massen für dentaltechnische, (dental)medizinische und verwandte Zwecke, welche einen thermoplastischen Kunststoff und übliche Zusatzstoffe umfasst, sowie Verfahren zu deren Herstellung und die Verwendung derselben.

Die Verwendung von Acryl- oder Methacrylsäureestern für (dental)medizinische und -technische Zwecke, insbesondere bei der Abnahme von Kieferabdrücken sowie zur Unterfütterung von Kunststoff-Zahnprothesen usw. ist bekannt. Es ist auch bereits bekannt, den Acryl- oder Methacrylsäureestern zu diesem Zweck Zusatzstoffe, wie Kunststoffe anderen Typus, Cellulosederivate, (natürliche) Harze (wie Copal, Sandarac), Paraffin, Wachs, Öl, Farbstoffe und Füllstoffe zuzufügen. DE-OS 27 18 017 beschreibt den Zusatz von Metallseife und/oder Metallsilicat zu derartigen plastischen bzw. harten Massen für dentaltechnische und (dental)medizinische Zwecke.

Die Acryl- oder Methacrylsäureester werden im allgemeinen in der Weise angewendet, dass ein Pulver, Perl- oder Splittergranulat eines Polymerisates oder Mischpolymerisates dieser Verbindungen in einem flüssigen Monomeren gelöst und diese Lösung gegebenenfalls unter Zusatz von Promotoren zur Erhärtung gebracht wird. Bei Verwendung derartiger Gemische ergeben sich jedoch gewisse Nachteile.

Während das Auflösen des pulvers in der Flüssigkeit verhältnismässig zeitraubend ist, bleiben bei Kaltpolymerisation im Pulver-Flüssigkeits-System Teile des Polymerisates ungelöst, wodurch Stabilität und Homogenität des Materials leiden. Hier ist es wünschenswert, eine Masse zu schaffen, die eine leichtere Verarbeitbarkeit gewährleistet, gegebenenfalls auf der Grundlage einer verbesserten Viskosität und Plastizität des Materials.

Die auf monomeren Methylmethacrylat enthaltenden Kaltpolymerisate gelten weiterhin als schleimhaut feindlich. Auch in dieser Beziehung ist es wünschenswert, eine verbesserte Dentalmasse zu schaffen, wodurch dieser Nachteil vermieden bzw. gemildert wird. Darüber hinaus können sich durch schnelles Auspolymerisieren von Kaltpolymerisaten im Munde Verbrennungen etc. ergeben. Bei der Herstellung von Unterfütterungen, ausgehend von monomerem Methylmethacrylat, weisen die erzeugten produkte nicht immer perfekt glatte Oberflächen auf, wodurch sie zum Teil speichelabsorbierend werden und dadurch durch die Mundmikroflora leichter angreifbar sind.

Der Erfindung liegt daher die Aufgabe zugrunde, eine Masse der eingangs genannten Art zu schaffen, die aufgrund ihrer verbesserten Viskosität eine leichtere und schnellere Verarbeitung gewährleistet, eine verbesserte mechanische Festigkeit besitzt und eine heilende Wirkung bei Druckstellen und Entzündungen im Mundbereich sowie bei Gliederprothesen entfaltet, glatte Flächen ausbildet, geruch- und geschmacklos sowie gut schleimhautverträglich ist, und eine optimale Passgenauigkeit über längere Zeiträume hin beibehält. Diese Masse soll durch geringfügige Variation im Bedarfsfall auf die jeweils gewünschte Konsistenz, z.B. hart oder elastisch oder weichbleibend, leicht einstellbar sein.

Die vorstehende Aufgabe wird gemäss der Erfindung durch Bereitstellung einer Masse der eingangs genannten Art gelöst, die dadurch gekennzeichnet ist, dass sie neben einem thermoplastichen Kunstsoff und üblichen Zusatzstoffen 0,05 bis 10 Gew.-% Jojobaöl enthält.

Jojobaöl, wie es gemäss der Erfindung verwendet wird, ist das aus der Jojobapflanze, die zur Familie der Buxaceae gehört, gewonnene und gereinigte Öl. Im weiteren soll der Begriff "Jojobaöl", wie er gemäss der Erfindung verwendet wird, auch Jojobawachs umfassen.

Es ist bevorzugt, das Jojobaöl zusammen mit den anderen Komponenten zur Herstellung von plastischen bzw. harten Massen gemäss der Erfindung in einer Konzentration von 0,I bis 5 Gew.-% zu verwenden.

Durch Zugabe von Jojobaöl zu der Masse zur Herstellung von plastischen bzw. harten Massen gemäss der Erfindung ist es möglich, die Verarbeitbarkeit, Zähigkeit, Härte und Festigkeit sowie die Oberflächeneigenschaften und die Haltbarkeit im Munde (keine Alterungserscheinungen, sowie Brüchigkeit, Geruchsbildung und unansehnliche Farbbildung zu verbessern, wobei die erfindungsgemässe Masse eine heilende Wirkung bei Druckstellen und Entzündungen im Mundbereich sowie bei Gliederprothesen entfaltet, und nicht poröse, nicht brüchige und nicht schrumpfende sowie insbesondere besonders gut schleimhautverträgliche Massen zu erhalten, die sich für die verschiedensten (dental)medizinischen und dentaltechnischen und andere Zwecke hervorragend eignen. Mit Hilfe der erfindungsgemässen Masse gelingt es insbesondere, je nach Wunsch, elastische, weichbleibende oder harte Abformmaterialien bzw. Unterfütterungen zu erzielen, wobei Härte bzw. Elastizität je nach Bedarf beliebig reguliert werden können. Insbesondere zeichnet sichdie erfindungsgemässe Masse dadurch aus, dass eine beliebig plastische oder harte dentaltechnische bzw. (dental)medizinische Masse erzielt werden kann, die ihre optimale Passganauigkeit und Viskosität bzw. Plastizität im Munde über lange Zeit aufrechterhält, eine heilende Wirkung bei Druckstellen und Entzündungen im Mundbereich sowie bei Gliederprothesen entfaltet und eine gute Schleimhautverträglichkeit besitzt.

Als Lösungs- bzw. Dispergiermittel für den Homo-, Co- bzw. Mischpolymeranteil kann der Methylester von Acryl- und/oder Methacrylsäure Verwendung finden, sowie bei der Heisspolymerisation von Acrylaten ohne Methylesterzusatz. Diese Ausführungsform der Erfindung kommt inbesondere für die Anwendung in Betracht, bei der eine Heisspolymerisierung vorgesehen ist, bzw. ein Auspolymerisieren im Mund des Patienten selbst unterbleibt. Eine besonders bevorzugte Masse gemäss der Erfindung weist jedoch keinen wesentlichen, oder besonders vorteilhaft, keinen Anteil an monomeren Acrylsäure- bzw. Methacrylsäuremethylestern auf, sondern es treten an dessen Stelle höher siedende Esterderivate der genannten Säuren. Hierbei wird insbesondere ein Gehalt von zumindest einem monomeren Acryl- und/oder Methacrylsäureester, der 6 bis 10 Kohlenstoffatome aufweist, in Betracht gezogen. Beispiele hierfür sind 2,3-Epoxypropyl-, n- bzw. t-Butyl-, n- bzw. Cyclhexyl-methacrylsäureester oder Mischungen dieser Monomeren. Ebenfalls in Betracht gezogen werden die analogen Ester der Acrylsäure, wobei es besonders zweckmässig sein kann, Abmischungen aus den genannten monomeren Acrylsäureestern und Methacrylsäureestern einzusetzen. Wenngleich der Einsatz von Esterderivaten mit insgesamt 6 bis 10 Kohlenstoffatomen bevorzugt ist, so können jedoch auch zumindest teilweise Esterderivate von Acryl- bzw. Methacrylsäure Verwendung finden, deren Gesamtkohlenstoffzahl unter bzw. über dem als vorteilhaft angeführten Bereich von 6 bis 10 Kohlenstoffatomen liegt, z.B. Methacrylsäure-dodecylester, Methacrylsäuretriethylenglykol-monoethylester, Methacrylsäureethylhexylester, etc. Ein besonders vorteilhaftes Monomeres stellt schliesslich ein Umsetzungsprodukt aus Glycidylmethacrylat und Bisphenol A dar.

Die Polymeren können grundsätzlich Polyacrylate und -methacrylate bzw. Misch- und/oder Copolymerisate hiervon darstellen. Daneben können auch gewisse Anteile von Kunststoffen anderen Typus, wie z.B. Polyvinylchlorid, Polyvinylacetat oder Polyvinylalkohol vorliegen. Bevorzugt ist ein Gemisch von Polymethacrylaten mit mittlerem Molekulagewicht. Unter diesen sind wiederum diejenigen besonders vorteilhaft, die gute Löslichkeitseigenschaften in den verwendeten Monomeranteilen und den zum Teil hierin enthaltenen Lösungsmittelanteilen aufweisen. Derartige Polymethylmethacrylat-Gemische mittleren Molekulargewichtes, die beispielsweise in Estern, Ketonen, chlorierten aliphatischen Kohlenwasserstoffen, cyclischen Ethern, etc., löslich sind und thermoplastische Eigenschaften aufweisen, befinden sich im Handel.

Bevorzugt stellt der thermoplastische Kunststoff vor dem Auspolymersieren somit ein Gemisch aus Monomeren und Polymeren bzw. Mischpolymeren von Acryl- und Methacrylsäureestern dar. Dieses Gemisch liegt bevorzugt als eine Lösung oder Paste vor.

Der Polymeranteil in der monomeren Lösung, die gegebenenfalls Zusätze enthält, kann innerhalb weiter Bereiche variieren. Überlicherweise werden etwa Polymeranteile zwischen 10 und 70 Gew.-%, bezogen auf die Gesamtzusammensetzung, vorliegen. Ein besonders bevorzugter Bereich des Gewichtsanteiles des Polymers beträgt 10 bis 40%, bezogen auf die Gesamtmasse, da hier besonders leicht flüssige bis pastenförmige und teigige Massen entstehen, die gegossen, gespritzt, mit der Spatel verteilt oder aus Druckgefässen als fliessende, teigige oder pastenförmige Massen herausgedrückt und verteilt werden können.

Den Lösungen oder dem Sirup, den Pasten oder angeteigten Massen aus Polymerisaten oder Mischpolymerisaten in dem Monomergemisch werden Metallseifen und/ oder Silicate, ein Katalysator und gegebenenfalls ein Beschleuniger zur Erhärtung zugesetzt.

Als Metallseifen haben sich Stearate, Laurate, Oxystearate, Palmitate, Montanate, Oleate oder Rizinoleate von Metalle, wie z.B. Aluminium, Magnesium oder Calcium als geeignet erwiesen. Bevorzugt sind die Erdalkaliseifen, wobei diese Metallseifen in feinverteiltem Zustand vorliegen sollen. Besonders bevorzugt ist die Verwendung von Magnesiumstearat und Calciumstearat. Die Zusatzmengen an Metallseife(n) bzw. deren Gemischen betragen vorteilhaft 0,1 bis 10 Gew.-%, berechnet auf die Gesamtmasse.

Als Silicate, deren zusätzliche Verwendung sich als günstig erwiesen hat, kommen in erster Linie Alkalisilicate in Betracht, insbesondere handelsübliche, lösliche Wasserglasverbindungen, wobei sowohl Natrium- wie Kaliumsilicate oder auch gemischte Alkalisilicate Verwendung finden können. Besonders gut geeignet ist Alkalialuminiumsilicat. Die Zusatzmenge an derartigem Silicat beträgt vorzugsweise 1,5 bis 5%, berechnet auf die Gesamtmenge der MasseBei diesen Wasserglaspräparaten sollte die sogenannte Ölzahl nicht über 26 liegen, wobei sich besonders eine Ölzahl von etwa 22 bewährt hat.

Im Falle besonderer Beanspruchung der plastischen bzw. harten Masse für dentaltechnische oder (dental)medizinische und verwandte Zwecke kann es zweckmässig sein, dass die erfindungsgemässen Massen einen Anteil an vernetzendem Agens enthalten. Unter den für die Vernetzung von Methacrylat bzw. Acrylat wirkenden, bekannten Stoffen, sind besonders olefinische Dimethylacrylate, wie Ethylen-dimethacrylat, Propylendimethacrylat, Polyethylenglykol-dimethacrylat, geeignet. Hierbei ist es zweckmässig, dass die gegebenenfalls eingesetzten Polyethylenglykoldimethacrylat-Vernetzer ein relativ niedriges Molekulargewicht aufweisen. Das Vernetzungsagens, das besonders geeignet ist, das spätere Auftreten von Spannungsrissen zu verhindern, kann beispielsweise in Mengen von 0.1 bis zu 10 Gew.-%, bezogen auf die Gesamtmasse, eingesetzt werden.

Das vernetzende Agens kann jedoch auch in höherer Menge eingesetzt werden und das monomere (Meth)acrylat teilweise ersetzen. Nach einer Ausführungsform der Erfindung, die für gewisse Einsatzzwecke als bevorzugt angesehen werden kann, wird der Monomeranteil sogar vollständig durch ein monomeres Vernetzungsagens ersetzt. Im letzteren Fall wäre somit der Polymeranteil ausschliesslich im monomeren Vernetzungsagens aufzulösen.

Nach einer bevorzugten Ausführungsform besteht die erfindungsgemässe Masse zur Herstellung von plastischen bzw. harten Massen für dentaltechnische und (dental)medizinische Zwecke aus einem Zwei-Komponenten-System, besonders bevorzugt aus einem Pulversystem und einem Flüssigsystem, wobei das Pulversystem den Polymerkunststoff und gegebenenfalls die Metallseife, Füllstoffe und Peroxid (Katalysator), und das. Flüssigsystem das monomere Acrylat, den Weichmacher, den Beschleuniger, sowie das Jojobaöl umfasst.

Im übrigen kann, um die Haltbarkeit der Massen gemäss der Erfindung zu erhöhen, denselben eine geringe Menge an Stabilisatoren oder Inhibitoren zugesetzt werden, die eine unbeabsichtigte Weiterpolymerisation der Lösung beim Aufbewahren verhindern. Hierfür kommen z.B. Phenolverbindungen, wie Aminophenole, Dibutylmethylphenol oder Butylhydroxyanisol oder auch Hydrochinon, Pyrogallol, Brenzkatechin in Betracht. Diese Hemmstoffe können in Mengen von etwa 2 bis 100 ppm der Masse zugesetzt werden.

Methacrylate haben meist einen typisch bitteren Nachgeschmack, der vorübergehend, z.B. beim Anpassen des mit Abformmaterial versehenen (Funktions)abdrucklöffels oder der Unterfütterung einer Prothese, störend wirken kann. Um diesen zu überbrücken, setzt man der Masse zweckmässig noch kohlenhydratefreie Süssstoffe, wie Cyclamate, oder aber antikariogene Zuckerzusatzstoffe auf Kohlenhydratbasis, z.B. Xylit, zu; besonders bewährt hat sich ein handelsübliches Gemisch aus Natriumcyclamat mit 10% Saccharin. Ausserdem kann die Lösung die üblichen pigmentzusätze enthalten.

Der zur Härtung der Kunststoffmasse verwendete Katalysator kann grundsätzlich in Pulverform zugesetzt werden. Vorteilhaft wird er jedoch in Form einer Lösung in einem Lösungsmittel angewendet, das vorzugsweise leicht löslich sein soll, da er sich auf diese Weise leicht und gleichmässig in der Acrylatlösung verteilen lässt. Als Katalysatoren oder Initiatoren verwendet man in bekannter Weise Peroxide, wie Wasserstoffperoxid, tert. Butylhydroperoxid, Cumolhydroperoxid, ferner Dialkyl- und Diarylperoxide, Ketonperoxide, Diacylperoxide, wie Dibenzoylperoxid, oder Peroxysäuren, ferner Azoverbindungen, wie Azo-di-isobuttersäurenitril und Azodicarbonamid, die gegebenenfalls in Lösungsmitteln, wie Dibutyl-phthalat, Methanol, Essigester, Aceton oder Methylethylketon gelöst, zur Anwendung kommen.

Bei Katalysatorsystemen, die selbsthärtend sind, d.h. ohne zusätzliche Anwendung von Wärme eine Aushärtung bewirken sollen, gehört zu dem eigentlichen Katalysator oder Initiator noch ein Beschleuniger oder Aktivator, der den Zerfall des Initiators und damit den Start der Polymerisation des Monomeren zum Polymeren bewirkt. Als solche Beschleuniger haben sich tert. Amine, Alkyl-, Alkylaryl- und Oxyalkylamine, ferner Reduktionsmittel, wie Sulfisäuren oder Dithionite bewährt, die etwa in Mengen von 1 bis 3% zugesetzt werden können. Anstelle der angeführten Katalysatorsysteme können naturgemäss auch alle anderen für die Polymerisation von Acrylaten bzw. Methacrylaten verwendbaren Systeme Anwendung finden. Als Beschleuniger kommt z.B. para-Toluolamin infrage, in Mengen zwischen 0,5 und 2%. Der Beschleuniger befindet sich erfindungsgemäss vorzugsweise im Sirup oder in der Paste oder in der Flüssigkeit selbst. Die Härtung kann aber auch in Abwesenheit von Beschleuniger z.B. durch Einwirkung von UV-Strahlung bewirkt werden. Bei der Heisspolymerisation wird die Härtung ohne Beschleuniger durch Wärmezufuhr erreicht.

Die erfindungsgemässen Massen werden bevorzugt auf die Weise hergestellt, dass unmittelbar vor Verwendung der Masse in ein Zwei-Komponenten-System, bestehend aus einem Pulversystem, welches das Polymere, gegebenenfalls Füllstoffe und Metallseifen, und Katalysator umfasst, und ein Flüssigsystem, bestehend aus dem monomeren Acrylat, welches gegebenenfalls Weichmacher, Beschleuniger und das erfindungsgemäss vorgesehene Jojobaöl umfasst, vermischt wird.

Bei der praktischen Anwendung der erfindungsgemässen Masse wird beispielsweise zur Unterfütterung einer Prothese, die Masse auf die vorher aufgeraute, zweckmässig mit einem Lösungsmittel angelöste Oberfläche der Prothese aufgetragen und in den Mund des Patienten eingesetzt, wo die Masse innerhalb weniger Minuten unter Druck und gegebenenfalls unter Luftabschluss völlig auspolymerisiert. Bei der Durchführung von Reparaturen von Zahnprothesen ist es z.B. vorteilhaft, die aufgetragene erfindungsgemässe Masse mit Glas- oder Cellophanpapier abzudecken, da die Aushärtung zweckmässig unter Luftabschluss erfolgen sollte.

Bei der Verwendung der erfindungsgemässen Massen in der (Dental)medizin bzw. Dentaltechnik ergibt sich der grosse Vorteil, dass nunmehr lediglich die fertige Polyacrylatlösung durch Zusatz einer Katalysatorlösung, vorzugsweise in Form eines Zwei-Komponenten-Systems aus einem Pulversystem und einem Flüssigsystem, in kürzester Zeit zur Erstarrung gebracht werden kann und auf diese Weise, ohne dass eine Schrumpfung oder Alterung des Materials im Laufe der Zeit eintritt, eine optimale Passgenauigkeit und Abformung durch das Abform- bzw. Unterfütterungsmaterial erzielt werden kann. Hierbei können je nach Wunsch elastische, weichbleibende oder harte Dentalmassen erzielt werden, die Auspolymerisation kann vollkommen im Munde des Patienten erfolgen, ohne dass auch bei Kaltpolymerisierung Verbrennungen, Verätzungen, Reizungen der Schleimhaut auftrete.

Die erfindungsgemässen Massen sind vor allem für dentale Zwecke, wie zur Herstellung eines individuell angepassten Funktionsabdrucklöffels mit der entsprechenden Abformungzur Herstellung einer Zahnprothese, als Abformmaterial und Unterfütterungsmaterial bei der Herstellung eines Zahnschutzes, insbesondere für Sportler, wie dies in der parallelen Patentanmeldung der Anmelderin, eingereicht am 19. Oktober 1983, beschrieben ist, allgemein als Abdruckmassen, bei Unterfütterungen und Reparaturen von Zahnprothesen geeignet. Sie können aber auch andere Anwendung finden, beispielsweise als Fingernagelersatz oder für die Otoplastik, die Reparatur von Hörgeräten, sowie für die Unterfütterung von Bein- und Armprothesen und dergleichen, als Hautersatz, zur Herstellung von Weich- und Knochenteilen in der Medizin sowie für kosmetische Korrekturen in der Medizin.

Ein besonderer Vorteil bei der Verwendung der erfindungsgemässen Massen besteht darin, dass aufgrund der einfachen Handhabung derselben diese auch in der Selbstmedikation, z.B. für Träger von Teil- und Totalprothesen, für kosmetische Korrekturen in der Selbstmedikation, wie z.B. Korrekturen der Lachfalten bei prothesenträgern, eingesetzt werden können.

Die folgenden Beispiele sollen die erfindungsgemässe Masse sowie das Verfahren zu deren Herstellung und deren Verwendung näher erläutern, ohne den Umfang der Erfindung zu beschränken.

Beispiel 1

In einem Mischgefäss mischt man 25 Gewichtsteile eines Copolymeren, bestehend aus 96 Gewichtsteilen Methylmethacrylat und 4 Gewichtsteilen Ethylacrylat, in 75 Gewichtsteilen Acrylsäurecyclohexylester, dem 1% P-Toluolamin und 2% 1,4-Butan-dioldimethacrylat zugesetzt sind. Statt des Cycloalkylesters kann man auch 65 Gewichtsteile eines Gemisches von Methacrylatsäure-tertiär-butylester und Methacrylsäuremethylester im Verhältnis von 1:1 oder 1:2 verwenden. Dem erhaltenen Sirup fügt man 2% eines handelsüblichen Alkalialuminiumsilicates, 2,5% Magnesiumstearat sowie 1% Jojobaöl zu.

Bei Gebrauch setzt man dieser Mischung aus einem Tropffläschchen eine Lösung von 5 Gewichtsteilen Benzoylperoxid in 25 Gewichtsteilen Dibutylphthalat zu. Nach geringer Zeit fängt die Masse an, zähflüssig zu werden, so dass man sie nun auf die vorbereitete Prothese auftragen und in den Mund des Patienten einsetzen kann. Nach wenigen Minuten erhärtet hier die Masse zu einer harten Unterfütterung.

Beispiel 2

50 Teile eines Mischpolymerisats, bestehend aus 96 Gewichtsteilen Methylmethacrylat und 4 Gewichtsteilen Ethylacrylat, werden in einem Gemisch aus 50 Teilen Methacrylsäure-tertiär-butylester, Methacrylsäuremethylester und Methacrylsäure-n-hexylester, die im Verhältnis von 1:1:1 gemischt sind, gelöst. Dieser Menge fügt man 3% Magnesiumstearat und 1% eines handelsüblichen Alkalialuminiumsilicats, 1% p-Toluolamin sowie 1% Jojobaöl zu. Wie in Beispiel 1 setzt man zur Verarbeitung dieser Masse eine Benzoyl-peroxidlösung tropfenweise hinzu und erhält in kurzer Zeit eine zähflüssige Masse, die sich gut auf der Prothese verteilen lässt und nach kurzer Zeit eine elastische Unterfütterung ergibt, die eine ausgezeichnete Passform aufweist, beständig ist und praktisch keine Schrumpfung zeigt.

Beispiel 3

Im folgenden wurde ein Zahnschutz für Sportler hergestellt, wobei von einem Funktionsabdrucklöffel, wie er in DE-OS 27 18 017 beschrieben ist, ausgegangen wurde. Dieser, aus thermoplastischem Material bestehende Funktionslöffel wird durch Eintauchen in warmes Wasser von 70°C erweicht und der Zahn- und Kiefersituation individuell durch leichtes Andrücken angepasst.

Im folgenden wird ein Abformmaterial/Unterfütterungsmaterial, bestehend aus 40 Gew.-% Polyacrylat, ca. 55 Gew.-% Methacrylsäurehexylester, 1 Gew.-% Calciumstearat und 1 Gew.-% Jojobaöl, zusammen mit üblichen Härtern und Beschleunigern, in den Funktionsabdrucklöffel eingefüllt, und dieser nochmals den Zahn- und Kieferverhältnissen im Munde angepasst. Das vorstehend genannte Abformmaterial/Unterfütterungsmaterial wurde aus einem Zwei-Komponenten-System hergestellt, das unmittelbar vor Anwendung desselben miteinander vermischt wurde Nach ca. 10 Minuten ist das elastische Abformmaterial/Unterfütterungsmaterial auspolymerisiert und bildet eine weiche Pufferzone zwischen dem relativ harten Material des Löffels und den Zähnen. Sofern der verwendete Funktionsabdrucklöffel mit Aussparungen im Aufbissbereich versehen ist, dringt darüber hinaus beim Andrücken des mit Abformmaterial/Unterfütterungsmaterial ausgefüllten Funktionsabdrucklöffels eine Teil dieses Abformmaterial/Unterfütterungsmaterials durch die Aussparungen hindurch, polymerisiert dort aus und bildet im weiteren eine weiche Pufferzone zwischen der unteren und oberen Zahnreihe. Da das Verfahren in einfacher Weise durchzuführen ist, kann ein derartiger Zahnschutz, wie er vorstehend beschrieben wird, auch vom Sportler selbst hergestellt werden.

In gleicher Weise kann das vorstehend beschriebene Material als Abformmaterial, das in einen bereits präliminär der Kiefersituation des Patienten angepassten Funktionsabdrucklöffel eingefüllt ist, dazu dienen, einen exakteren, modellgetreuen, nicht schrumpfenden Abdruck zu erzielen. Es ist besonders vorteilhaft gemäss der Erfindung, dass bei Verwendung eines Funktionsabdrucklöffels aus Acrylatkunststoff zusammen mit einem auf der Basis von Acrylat aufgebautem Abformmaterial die Verwendung eines Adhäsivs nicht erforderlich ist, wie dies bei den handelsüblichen Abformmaterialien notwendig ist. Dadurch kann auf einfache Weise ein verbesserter Funktionsabdrucklöffel bzw. Situationsabdruck zur Herstellung einer Prothese bzw. Teilprothese hergestellt werden, wodurch insbesondere auch besser sitzende und passgenauere Prothesen hergestelt werden können.

**Patentansprüche für die Vertragsstaaten: BE CH DE FR GB IT LI LU NL SE**

1. Masse zur Herstellung von plastischen bzw. harten Massen für dentaltechnische, (dental)medizinische und verwandte Zwecke, welche einen thermoplastischen Kunststoff und übliche Zusatzstoffe umfasst, dadurch gekennzeichnet, dass die genannte Masse ausserdem 0,05 bis 10 Gew.-% Jojobaöl enthält.

2. Masse gemäss Anspruch 1, dadurch gekennzeichnet, dass die Konzentration an Jojobaöl 0,1 bis 5 Gew.-% beträgt.

3. Masse gemäss Anspruch 1 und 2, dadurch gekennzeichnet, dass der thermoplastische Kunststoff vor dem Auspolymerisieren aus einem Gemisch von Monomeren und Polymeren bzw. Mischpolymeren von Acryl- und Methacrylsäureestern besteht.

4. Masse gemäss Anspruch 3, dadurch gekennzeichnet, dass der thermoplastische Kunststoff monomeres und/oder polymeres Methylmethacrylat umfasst.

5. Masse gemäss Anspruch 3, dadurch gekennzeichnet, dass der thermoplastische Kunststoff einen Acryl- oder Methacrylester mit insgesamt 6 bis 10 Kohlenstoffatomen oder ein Gemisch derselben umfasst.

6. Masse gemäss Anspruch 5, dadurch gekennzeichnet, dass der Ester einen Hexylester darstellt.

7. Masse gemäss Anspruch 3, dadurch gekennzeichnet, dass der Acrylat-Kunststoff im wesentlichen kein Methylmethacrylat umfasst.

8. Masse gemäss Anspruch 3, dadurch gekennzeichnet, dass der thermoplastische Kunststoff vor dem Auspolymerisieren eine Lösung oder Paste aus Polymer(en) bzw. Mischpolymeren in monomerem Acryl- und/oder Methacrylsäureester darstellt.

9. Masse gemäss Anspruch 8, dadurch gekennzeichnet, dass der Gewichtsanteil des Polymers 10 bis 40%, bezogen auf die Gesamtmasse, beträgt.

10. Masse gemäss Anspruch 1, dadurch gekennzeichnet, dass diese übliche Härter und Beschleuniger umfasst.

11. Masse gemäss Anspruch 10, dadurch gekennzeichnet, dass diese eine Komponente eines Zwei-Komponenten-Beschleuniger- bzw. -Härtungssystems umfasst.

12. Masse gemäss Anspruch 1, dadurch gekennzeichnet, dass diese einen Anteil an Metallseife und/oder Silicat umfasst.

13. Masse gemäss Anspruch 12, dadurch gekennzeichnet, dass die Metallseife in einer Konzentration von 0,1 bis 10 Gew.-% vorliegt.

14. Masse gemäss Anspruch 12, dadurch gekennzeichnet, dass die Metallseife Calcium- und/oder Magnesiumstearat darstellt.

15. Masse gemäss Anspruch 12, dadurch gekennzeichnet, dass das Silicat in einer Konzentration von 1,5 bis 5 Gew.-% vorliegt.

16. Masse gemäss Anspruch 15, dadurch gekennzeichnet, dass das Silicat ein Alkalialuminiumsilicat darstellt.

17. Masse gemäss Anspruch 1, dadurch gekennzeichnet, dass diese ein vernetzendes Agens enthält.

18. Masse gemäss Anspruch 17, dadurch gekennzeichnet, dass das vernetzende Agens ein olefinisches Dimethacrylat oder ein (Poly)ethylenglycoldimethacrylat darstellt, das dem monomeren (Meth)acrylat zugefügt ist oder den Monomeranteil ausschliesslich darstellt.

19. Masse nach einem oder mehreren der vorangehenden Ansprüche, dadurch gekennzeichnet, dass diese aus einem Pulversystem und einem Flüssigsystem besteht, wobei das Pulversystem den Polymerkunststoff und gegebenenfalls die Metallseife, Füllstoffe und Katalysator, und das Flüssigsystem das monomere Acrylat, den Weichmacher, den Beschleuniger sowie das Jojobaöl umfasst.

20. Verfahren zur Herstellung der Masse nach einem der Ansprüche 1 bis 19, dadurch gekennzeichnet, dass ein Zwei-Komponenten-System, bestehend aus einem Pulversystem, welches das Polymere, gegebenenfalls Füllstoffe, Metallseifen und Katalysator umfasst, und ein Flüssigsystem, bestehend aus dem monomeren Acrylat, welches gegebenenfalls Weichmacher und Beschleuniger und Jojobaöl umfasst, vermischt werden.

21. Verwendung der Masse nach einem oder mehreren der Ansprüche 1 bis 19 zur Herstellung von dentaltechnischen und (dental)medizinischen Abform- und Unterfütterungsmaterialien.

22. Verwendug der Masse nach einem oder mehreren der Ansprüche 1 bis 19 zur Herstellung von Unterfütterungen von Gliederprothesen.

23. Verwendung der Masse nach einem oder mehreren der Ansprüche 1 bis 19 als Abformmaterial zur Herstellung eines Zahnschutzes, insbesondere für Sportler, sowie bei der Herstellung eines individuell angepassten (Funktions)abdrucklöffels.

**Patentansprüche für den Vertragsstaat: AT**

1. Verfahren zur Herstellung einer Zubereitung für plastische bzw. harte Massen für dentaltechnische, (dental)medizinische und verwandte Zweck, welche einen thermoplastischen Kunststoff und übliche Zusatzstoffe umfasst, dadurch gekennzeichnet dass die genannte Masse ausserdem 0,05 bis 10 Gew.-% Jojobaöl enthält.

2. Verfahren gemäss Anspruch 1, dadurch gekennzeichnet, dass man das Jojobaöl in einer Konzentration von 0,1 bis 5 Gew.-% zumischt.

3. Verfahren gemäss Anspruch 1 und 2, dadurch gekennzeichnet, dass der thermoplastische Kunststoff vor dem Auspolymerisieren aus einem Gemisch von Monomeren und Polymeren bzw. Mischpolymeren von Acryl- und Methacrylsäureestern ausgewählt wird.

4. Verfahren gemäss Anspruch 3, dadurch gekennzeichnet, dass man als thermoplastischen Kunststoff monomeres und/oder polymeres Methylmethacrylat verwendet.

5. Verfahren gemäss Anspruch 3, dadurch gekennzeichnet, dass man als thermoplastischen Kunststoff einen Acryl- oder Methacrylester mit insgesamt 6 bis 10 Kohlenstoffatomen oder ein Gemisch derselben verwendet.

6. Verfahren gemäss Anspruch 5, dadurch gekennzeichnet, dass man als Ester einen Hexylester verwendet.

7. Verfahren gemäss Anspruch 3 dadurch gekennzeichnet, dass ein Acrylatkunststoff verwendet wird, der im wesentlichen kein Methylmethacrylat umfasst.

8. Verfahren gemäss Anspruch, 3, dadurch gekennzeichnet, dass als thermoplastischer Kunststoff vor dem Auspolymerisieren eine Lösung oder Paste aus Polymer(en) bzw. Mischpolymeren in monomerem Acryl- und/oder Methacrylsäureester verwendet wird.

9. Verfahren gemäss Anspruch 8, dadurch gekennzeichnet, dass das Polymer in einem Gewichtsanteil von 10 bis 40%, bezogen auf die Gesamtmasse, verwendet wird.

10. Verfahren gemäss Anspruch 1, dadurch gekennzeichnet, dass der Zubereitung übliche Härter und Beschleuniger zugegeben werden.

11. Verfahren gemäss Anspruch 10, dadurch gekennzeichnet, dass der Zubereitung eine Komponente eines Zwei-Komponenten-Beschleuniger- bzw. Härtungssystems zugegeben wird.

12. Verfahren gemäss Anspruch 1, dadurch gekennzeichnet, dass der Zubereitung ein Anteil an Metallseife umd/oder Silicat zugegeben wird.

13. Verfahren gemäss Anspruch 12, dadurch gekennzeichnet, dass die Metallseife in einer Konzentration von 0,1 bis 10 Gew.-% zugegeben wird.

14. Verfahren gemäss Anspruch 12, dadurch gekennzeichnet, das als Metallseife Calcium- und/oder Magnesiumstearat zugegeben wird.

15. Verfahren gemäss Anspruch 12, dadurch gekennzeichnet, dass das Silicat in einer Konzentration von 1,5 bis 5 Gew.-% zugegeben wird.

16. Verfahren gemäss 15, dadurch gekennzeichnet, dass als Silicat ein Alkalialuminiumsilicat zugegeben wird.

17. Verfahren gemäss Anspruch 1, dadurch gekennzeichnet, dass der Zubereitung ein vernetzendes Agens zugegeben wird.

18. Verfahren gemäss Anspruch 17, dadurch gekennzeichnet, dass als vernetzendes Agens ein olefinisches Dimethylacrylat oder ein (Poly)ethylenglycoldimethacrylat, das dem monomeren (Meth)acrylat zugefügt ist oder den Monomeranteil ausschliesslich darstellt, zugegeben wird.

19. Verfahren nach einem oder mehreren der vorangehenden Ansprüche, dadurch gekennzeichnet, dass ein Pulversystem und ein Flüssigsystem hergestellt wird, wobei das Pulversystem den Polymerkunststoff und gegebenenfalls die Metallseife, Füllstoffe und Katalysator, und das Flüssigsystem das monomere Acrylat, den Weichmacher, den Beschleuniger sowie das Jojobaöl umfasst.

20. Verfahren zur Herstellung der Masse nach einem der Ansprüche 1 bis 19, dadurch gekennzeichnet, dass ein Zwei-Komponenten-System, bestehend aus einem Pulversystem, welches das Polymere, gegebenenfalls Fullstoffe, Metallseifen und Katalysator umfasst, und ein Flüssigsystem, bestehend aus dem monomeren Acrylat, welches gegebenenfalls Weichmacher und Beschleuniger, und Jojobaöl umfasst, vermischt werden.

21. Verwendung der Masse nach einem oder mehreren der Ansprüche 1 bis 19 zur Herstellung von dentaltechnischen und (dental)medizinischen Abformund Unterfütterungsmaterialien.

22. Verwendung der Masse nach einem oder mehreren der Ansprüche 1 bis 19 zur Herstellung von Unterfütterungen von Gliederprothesen.

23. Verwendung der Masse nach einem oder mehreren der Ansprüche 1 bis 19 als Abformmaterial zur Herstellung eines Zahnschutzes, insbesondere für Sportler, sowie bei der Herstellung eines individuell angepassten (Funktions)abdrucklöffels.

**Revendications pour les Etats contractants: BE CH DE FR GB IT LI LU NL SE**

1. Composition pour la production de matériaux plastiques durs pour technique dentaires, médecine (dentaire) et utilisation apparentée, comprenant une matière thermoplastique et des additifs usuels, caractérisée en ce que la composition citée contient en outre de 0,05 à 10% en poids d'huile de jujube.

2. Composition selon la revendication 3, caractérisée en ce que la concentration en huile de jujube est de 0,1 à 5% en poids.

3. Composition selon la revendication 1 et 2, caractérisée en ce qu'avant polymérisation, la matière thermoplastique se compose d'un mélange de monomères et de polymères, respectivement de copolymères d'esters d'acide acrrylique et méthacrylique.

4. Composition selon la revendication 3, caractérisée en ce que la matière thermoplastique contient un monomère et/ou un polymère de méthylméthacrylate.

5. Composition selon la revendication 3, caractérisée en ce que la matière thermoplastique contient un ester acrylique ou méthacrylique comprenant au total 6 à 10 atomes de carbone, ou un mélange de ceux-ci.

6. Composition selon la revendication 5, caractérisée en ce que l'ester et un hexylester.

7. Composition selon la revendication 3, caractérisée en ce que la matière plastique acrylique ne comprend pratiquement pas de méthylméthacrylate.

8. Composition selon la revendication 3, caractérisée en ce que la matière thermoplastique constitue avant polymérisation une solution ou une pâte composée de polymère(s), respectivement de copolymères dans un monomère d'ester d'acide acrylique et/ou d'acide méthacrylique.

9. Composition selon la revendication 8, caractérisée en ce que la proportion en poids du polymère est de 10 à 40% de la masse totale.

10. Composition selon la revendication 1, caractérisée en ce qu'elle contient des durcisseurs et des accélérateurs usuels.

11. Composition selon la revendication 10, caractérisée en ce qu'elle contient un composant d'un accélérateur à deux composants ou d'un système de durcissement à deux composants.

12. Composition selon la revendication 1, caractérisée en ce qu'elle contient une proportion de savon métallique et/ou de silicate.

13. Composition selon la revendication 12, caractérisée en ce que le savon métallique est présent en une concentration allant de 0,1 à 10% en poids.

14. Composition selon la revendication 12, caractérisée en ce que le savon métallique est un stéarate de calcium et/ou de magnésium.

15. Composition selon la revendication 12, caractérisée en ce que le silicate est présent en une concentration allant de 1,5 à 5% en poids.

16. Composition selon la revendication 15, caractérisée en ce que le silicate est un silicate d'alcaloaluminium.

17. Composition selon la revendication 1, caractérisée en ce qu'elle contient un agent réticulant.

18. Composition selon la revendication 17, caractérisée en ce que l'agent réticulant est un diméthacrylate oléfinique ou un (poly)éthylène-glycoldiméthacrylate, qui est adjoint au monomère d'acrylate ou de méthacrylate, ou qui constitue exclusivement la proportion en monomère.

19. Composition selon une ou plusieurs des revendications précédentes 1, caractérisée en ce qu'elle se compose d'un système en poudre et d'un système liquide, le système en poudre contenant la matière plastique polymère et, le cas échéant, le savon métallique, des charges et un catalyseur, et que le système liquide contient le monomère d'acrylate, le plastifiant, l'accélérateur, ainsi que l'huile de jujube.

20. Procédé de fabrication de la composition selon l'une des revendications 1 à 19, caractérisé en ce qu'on procède au mélange d'un système à deux composants, constitué d'un système en poudre, qui contient le polymère, le cas échéant des charges, le savon métallique et le catalyseur, et d'un système liquide, constitué du monomère d'acrylate, qui contient le cas échéant un plastifiant et un accélérateur, et de l'huile de jujube.

21. Utilisation de la composition selon une ou plusieurs des revendications 1 à 19, pour la production de matériaux de surmoulage et de sous-garnissage pour technique dentaire et médecine (dentaire).

22. Utilisation de la composition selon une ou plusieurs des revendications 1 à 19, pour la production de sous-garnissage de prothèses d'organes.

23. Utilisation de la composition selon une ou plusieurs des revendications 1 à 19, en tant que matériau de surmoulage pour la protection d'une protection de dent, en particulier pour sportifs, ainsi que pour la production d'une cuiller à empreinte (fonctionnelle), adaptée à un individu.

**Revendications pour l'Etat contractant: AT**

1. Procédé d'application d'une préparation pour matériaux plastiques durs pour technique dentaires, médecine (dentaire) et utilisation apparentée, comprenant une matière thermoplastique et des additifs usuels, caractérisée en ce que la composition citée contient en outre de 0,05 à 10% en poids d'huile de jujube.

2. Procédé selon la revendication 1, caractérisée en ce que l'on mélange l'huile de jujube à une concentration comprise entre 0,1 et 5% en poids.

3. Procédé selon la revendication 1 et 2, caractérisée en ce qu'avant polymérisation, la matière thermoplastique se compose d'un mélange de monomères et de polymères, respectivement de copolymères d'esters d'acide acrrylique et méthacrylique.

4. Procédé selon la revendication 3, caractérisée en ce qu'on utilise comme matière thermoplastique un monomère et/ou un polymère de méthylméthacrylate.

5. Procédé selon la revendication 3, caractérisée en ce qu'on utilise comme matière thermoplastique un ester acrylique ou méthacrylique comprenant au total 6 à 10 atomes de carbone, ou un mélange de ceux-ci.

6. Procédé selon la revendication 5, caractérisée en ce qu'on utilise comme ester un hexylester.

7. Procédé selon la revendication 3, caractérisée en ce qu'on utilise une matière plastique à base d'acrylate qui ne comprend pratiquement pas de méthylméthacrylate.

8. Procédé selon la revendication 3, caractérisée en ce que la matière thermoplastique constitue avant polymérisation une solution ou une pâte composée de polymère(s), respectivement de copolymères, dans un monomère d'ester d'acide acrylique et/ou d'acide méthacrylique.

9. Procédé selon la revendication 8, caractérisée en ce que la proportion en poids du polymère est de 10 à 40% calculé sur la masse totale.

10. Procédé selon la revendication 1, caractérisée en ce qu'on ajoute à la préparation des durcisseurs et des accélérateurs usuels.

11. Procédé selon la revendication 10, caractérisée en ce qu'on ajoute à la préparation un composant d'un accélérateur à deux composants ou d'un système de durcissement à deux composants.

12. Procédé selon la revendication 1, caractérisée en ce qu'on ajoute à la préparation une proportion de savon métallique et/ou de silicate.

13. Procédé selon la revendication 12, caractérisée en ce que le savon métallique est ajouté en une concentration allant de 0,1 à 10% en poids.

14. Procédé selon la revendication 12, caractérisée en ce qu'on ajoute comme savon métallique un stéarate de calcium et/ou de magnésium.

15. Procédé selon la revendication 12, caractérisée en ce que le silicate est ajouté en une concentration allant de 1,5 à 5% en poids.

16. Procédé selon la revendication 15, caractérisée en ce que le silicate ajouté est un silicate d'alcaloaluminium.

17. Procédé selon la revendication 1, caractérisée en ce qu'on ajoute à la préparation un agent réticulant.

18. Procédé selon la revendication 17, caractérisée en ce que l'agent réticulant ajouté est un diméthacrylate oléfinique ou un (poly)éthylène-glycoldiméthacrylate, qui est adjoint au monomère d'acrylate ou de méthacrylate, ou qui constitue exclusivement la proportion en monomère.

19. Procédé selon une ou plusieurs des revendications précédentes 1, caractérisée en ce qu'on fabrique un système en poudre et un système liquide, le système en poudre contenant la matière plastique polymère et, le cas échéant, le savon métallique, des charges et un catalyseur, et le système liquide contenant le monomère d'acrylate, le plastifiant, l'accélérateur, ainsi que l'huile de jujube.

20. Procédé de fabrication de la composition selon l'une des revendications 1 à 19, caractérisé en ce qu'on procède au mélange d'un système à deux composants, constitué d'un système en poudre, qui contient le polymère, le cas échéant des charges, le savon métallique et le catalyseur, et d'un système liquide, constitué du monomère d'acrylate, qui contient le cas échéant un plastifiant et un accélérateur, et de l'huile de jujube.

21. Utilisation de la composition selon une ou plusieurs des revendications 1 à 19, pour la production de matériaux de surmoulage et de sous-garnissage pour technique dentaire et médecine (dentaire).

22. Utilisation de la composition selon une ou plusieurs des revendications 1 à 19, pour la production de sous-garnissage de prothèses d'organes.

23. Utilisation de la composition selon une ou plusieurs des revendications 1 à 19, en tant que matériau de surmoulage pour la protection d'une protection de dent, en particulier pour sportifs, ainsi que pour la production d'une cuiller à empreinte (fonctionnelle), adaptée à un individu.

## Claims for the Contracting States: BE CH DE FR GB IT LI LU NL SE

1. Composition for the preparation of plastic or hard materials for dental technical, (dental) medical and related purposes, which includes a thermoplastic material and ordinary additives, characterised in that the said composition also contains 0.05 to 10 wt.% jojoba oil.

2. Composition according to claim 1, characterised in that the concentration of jojoba oil is 0.1 to 5 wt.%.

3. Composition according to claims 1 and 2, characterised in that the thermoplastic material before polymerisation consists of a mixture of monomers and polymers or copolymers of acrylic and methacrylic acid esters.

4. Composition according to claim 3, characterised in that the thermoplastic material includes monomeric and/or polymeric methyl methacrylate.

5. Composition according to claim 3, characterised in that the thermoplastic material includes an acrylic or methacrylic ester with a total of 6 to 10 carbon atoms or a mixture thereof.

6. Composition according to claim 5, characterised in that the ester is a hexyl ester.

7. Composition according to claim 3, characterised in that the acrylate plastic essentially contains no methyl methacrylate.

8. Composition according to claim 3, characterised in that the thermoplastic material before polymerisation is a solution or paste of polymer(s) or copolymers in monomeric acrylic and/ or methacrylic acid ester.

9. Composition according to claim 8, characterised in that the proportion by weight of polymer is 10 to 40%, based on the total composition.

10. Composition according to claim 1, characterised in that it contains ordinary hardeners and accelerators.

11. Composition according to claim 10, characterised in that it contains one component of a two-component accelerator or hardening system.

12. Composition according to claim 1, characterised in that it contains a proportion of metal soap and/or silicate.

13. Composition according to claim 12, characterised in that the metal soap is present in a concentration of 0.1 to 10 wt.%.

14. Composition according to claim 12, characterised in that the metal soap is calcium and/or magnesium stearate.

15. Composition according to claim 12, characterised in that the silicate is present in a concentration of 1.5 to 5 wt.%.

16. Composition according to claim 15, characterised in that the silicate is an alkali aluminium silicate.

17. Composition according to claim 1, characterised in that it contains a crosslinking agent.

18. Composition according to claim 17, characterised in that the crosslinking agent is an olefinic dimethacrylate or a (poly)ethylene glycol dimethacrylate which is added to the monomeric (meth)acrylate or constitutes the monomeric portion exclusively.

19. Composition according to one or more of the preceding claims, characterised in that it consists of a powder system and a liquid system, wherein the powder system includes the polymeric plastic and optionally the metal soap, fillers and catalyst, and the liquid system includes the monomeric acrylate, the softener, the accelerator and the jojoba oil.

20. Composition for the preparation of the composition according to any of claims 1 to 19, characterised in that a two-component system consisting of a powder system, which includes the polymer, optionally fillers, metal soaps and catalyst, and a liquid system consisting of the monomeric acrylate which includes optionally softeners and accelerators and jojoba oil, is mixed.

21. Use of the composition according to one or more of claims 1 to 19 for the preparation of dental technical and (dental) medical moulding and lining materials.

22. Use of the composition according to one or more of claims 1 to 19 for the preparation of linings of artificial limbs.

23. Use of the composition according to one or more of claims 1 to 19 as a moulding material for the manufacture of a gum shield, in particular for sportsmen, and in the manufacture of an individually fitted (functional) impression tray.

## Claims for the Contracting State: AT

1. Process for the production of a preparation for plastic or hard compositions for dental technical, (dental) medical and related purposes, which includes a thermoplastic material and ordinary additives, characterised in that the said composition also contains 0.05 to 10 wt.% jojoba oil.

2. Process according to claim 1, characterised in that the jojoba oil is added in a concentration of 0.1 to 5 wt.%.

3. Process according to claims 1 and 2, characterised in that the thermoplastic material before polymerisation is selected from a mixture of monomers and polymers or copolymers of acrylic and methacrylic acid esters.

4. Process according to claim 3, characterised in that monomeric and/or polymeric methyl methacrylate is used as the thermoplastic material.

5. Process according to claim 3, characterised in that an acrylic or methacrylic ester with a total of 6 to 10 carbon atoms or a mixture thereof is used as the thermoplastic material.

6. Process according to claim 5, characterised in that a hexyl ester is used as the ester.

7. Process according to claim 3, characterised in that an acrylate plastic is used, which essentially contains no methyl methacrylate.

8. Process according to claim 3, characterised in that a solution or paste of polymer(s) or copolymers in monomeric acrylic and/or methacrylic acid ester is used as the thermoplastic material before polymerisation.

9. Process according to claim 8, characterised in that the polymer is used in a proportion by weight of 10 to 40%, based on the total composition.

10. Process according to claim 1, characterised in that ordinary hardeners and accelerators are added to the preparation.

11. Process according to claim 10, characterised in that one component of a two-component accelerator or hardening system is added to the preparation.

12. Process according to claim 1, characterised in that a proportion of metal soap and/or silicate is added to the preparation.

13. Process according to claim 12, characterised in that the metal soap is added in a concentration of 0.1 to 10 wt.%.

14. Process according to claim 12, characterised in that calcium and/or magnesium stearate is added as the metal soap.

15. Process according to claim 12, characterised in that the silicate is added in a concentration of 1.5 to 5 wt.%.

16. Process according to claim 15, characterised in that an alkali aluminium silicate is added as the silicate.

17. Process according to claim 1, characterised in that a crosslinking agent is added to the preparation.

18. Process according to claim 17, characterised in that an olefinic dimethyl acrylate or a (poly)ethylene glycol dimethacrylate which is added to the monomeric (meth)acrylate or constitutes the monomeric portion exclusively, is added as the crosslinking agent.

19. Process according to one or more of the preceding claims, characterised in that a powder system and a liquid system is prepared, wherein the powder system includes the polymeric plastic and optionally the metal soap, fillers and catalyst, and the liquid system includes the monomeric acrylate, the softener, the accelerator and the jojoba oil.

20. Process for the preparation of the composition according to any of claims 1 to 19, characterised in that a two-component system consisting of a powder system, which includes the polymer, optionally fillers, metal soaps and catalyst, and a liquid system consisting of the monomeric acrylate which optionally includes softeners and accelerators, and jojoba oil, is mixed.

21. Use of the composition according to one or more of claims 1 to 19 for the preparation of dental technical and (dental) medical moulding and lining materials.

22. Use of the composition according to one or more of claims 1 to 19 for the preparation of linings of artificial limbs.

23. Use of the composition according to one or more of claims 1 to 19 as a moulding material for the manufacture of a gum shield, in particular for sportsmen, and in the manufacture of an individually fitted (functional) impression tray.